# EUROPEAN PATENT SPECIFICATION

(11) **EP 4 194 852 B1**
(45) Date of publication and mention of the grant of the patent: **19.02.2025**
(21) Application number: 22212951.2
(22) Date of filing: 12.12.2022
(51) Int. Cl.: G01N 33/00

(54) **CALIBRATING OR VERIFYING AN OPERATION OF A VOLATILE ORGANIC COMPOUND SENSOR USING CARBON MONOXIDE**
KALIBRIERUNG ODER ÜBERPRÜFUNG EINES BETRIEBS EINES SENSORS FÜR FLÜCHTIGE ORGANISCHE VERBINDUNGEN MIT KOHLENMONOXID
ÉTALONNAGE OU VÉRIFICATION D'UN FONCTIONNEMENT D'UN CAPTEUR DE COMPOSÉ ORGANIQUE VOLATIL À L'AIDE DE MONOXYDE DE CARBONE

(30) Priority: 10.12.2021 US 202163288132 P
(43) Date of publication of application: 14.06.2023
(73) Proprietor: Carrier Corporation, Palm Beach Gardens, FL 33418 (US)
(72) Inventor: BAILEY, Callum, Bradenton, 34202 (US); PATEL, Vipul, Bradenton, 34202 (US)
(74) Representative: Dehns

(56) References cited:
- US-A1- 2020 116 614
- US-B2- 8 907 803
- M. GROSSKLOS: "Use of VOC sensors for air quality control of building ventilation systems", JOURNAL OF SENSORS AND SENSOR SYSTEMS, vol. 4, no. 1, 8 May 2015 (2015-05-08), pages 159 - 168, XP055529645, DOI: 10.5194/jsss-4-159-2015
- BAUR TOBIAS ET AL: "Field Study of Metal Oxide Semiconductor Gas Sensors in Temperature Cycled Operation for Selective VOC Monitoring in Indoor Air", ATMOSPHERE, vol. 12, no. 5, 1 January 2021 (2021-01-01), Basel, pages 647, XP093035895, ISSN: 2073-4433, DOI: 10.3390/atmos12050647

## Description

### BACKGROUND

The present invention relates to gas detectors and, more particularly, to mass and force indicators in diagnostics and output correction for metal oxide and electrochemical gas sensors in gas detectors.

Air pollution is the presence of substances in the atmosphere that are harmful to the health of humans and other living beings, or cause damage to the climate or to materials. There are many different types of air pollutants, such as gases (including ammonia, carbon monoxide, sulfur dioxide, nitrous oxides, methane, carbon dioxide and chlorofluorocarbons), particulates (both organic and inorganic) and biological molecules. These pollutants may be both inside (e.g., in residential and commercial buildings) and outside. Volatile organic compounds (VOCs) are a common indoor air pollutant, and have been linked to adverse health effects. As such, monitoring their levels with VOC sensors forms an important component of indoor air quality (IAQ) monitoring for ensuring a healthy building and a healthy living or work environment. A number of technologies have been developed to monitor VOCs, including metal oxide and electrochemical sensors. However, to ensure accurate monitoring, the sensor operation may need to be verified both at the end of manufacturing and in the field (i.e., field calibration).

M. Großklos: "Use of VOC sensors for air quality control of building ventilation systems", Journal of Sensors and Sensor Systems, vol. 4, no. 1, 8 May 2015, pages 159-168 discloses the calibration of VOC sensors using carbon monoxide.

Baur Tobias et al: "Field Study of Metal Oxide Semiconductor Gas Sensors in Temperature Cycled Operation for Selective VOC Monitoring in Indoor Air", Atmosphere, vol. 12, no. 5, 1 January 2021, page 647 discloses the calibration of VOC sensors using various calibrants.

US 8907803 B2 discloses systems and methods for continuously monitoring residential air quality.

US 2020/116614 A1 discloses a method of evaluating manhole events based on mass flow rates of gaseous components.

### BRIEF DESCRIPTION

Viewed a first aspect of the invention, there is provided a method of calibrating a detector including a volatile organic compound (VOC) sensor that is sensitive to carbon monoxide (CO), and a CO sensor that is previously calibrated, as claimed in claim 1.

Optionally, the assembling of the VOC sensor is executed and completed in a manufacturing facility.

Optionally, the VOC sensor includes at least one of a metal oxide sensor and an electrochemical sensor.

Optionally, the CO sensor is configured to determine a verified amount of CO the detector is exposed to, the verified amount being used to calibrate the VOC sensor.

Optionally, the calibrating of the VOC sensor includes exposing the VOC sensor to the CO for at least a relatively short exposure time of about 4-15 minutes.

Optionally, the CO has about a 400 PPM concentration during the relatively short exposure time.

Optionally, the method further includes re-calibrating the VOC sensor using the CO as the calibrant following the field deployment.

Optionally, the re-calibrating is periodic, regularly scheduled or scheduled in response to a malfunction.

Additional features and advantages are realized through the techniques of the present invention. Other embodiments and aspects of the invention are described in detail herein and are considered a part of the claimed technical concept. For a better understanding of the invention with the advantages and the features, refer to the description and to the drawings.

### BRIEF DESCRIPTION OF THE DRAWINGS

For a more complete understanding of the present invention, reference is now made to the following brief description, taken in connection with the accompanying drawings and detailed description, wherein like reference numerals represent like parts:
FIG. 1 is a flow diagram illustrating an exemplary method of calibrating a detector that includes a volatile organic compound (VOC) sensor and a carbon monoxide (CO) sensor in accordance with embodiments;
FIG. 2 is a schematic illustration of a manufacturing facility and a field deployment of a VOC sensor in accordance with embodiments
FIG. 3 is a graphical depiction of an exemplary calibration sequence of a VOC sensor in accordance with embodiments; and
FIG. 4 is a flow diagram illustrating an exemplary method of calibrating a detector that includes a VOC sensor and a CO sensor in accordance with embodiments.

### DETAILED DESCRIPTION

Volatile organic compounds (VOCs) are a common air pollutant and monitoring their levels with VOC sensors forms an important component of indoor air quality (IAQ) monitoring for ensuring a healthy building and a healthy living or work environment. A number of technologies have been developed to monitor VOCs, including metal oxide and electrochemical sensors. In these cases, sensor operations need to be verified at the end of the manufacturing line. Additionally, sensors may need field calibration once they are deployed and then over time to monitor degradation.

As will be described below, carbon monoxide (CO) is used to verify operations of VOC sensors at the end of the production line. Metal oxide sensors, some electrochemical sensors and additional VOC sensing technologies may be sensitive to CO in addition to conventional calibrant VOC gases such as ethanol. VOC sensor functionality verification can be performed at the same time as CO calibration processes for combination systems featuring CO detectors or alarms that also include a VOC sensor, providing a manufacturing efficiency. Furthermore, periodic field calibrations of the VOC sensor can be performed using CO gas as the calibrant and may allow for CO and VOC combination alarm and sensor systems to be calibrated in one step (e.g., via a technician using a smoke detector test kit that emits a controlled amount of CO).

It should be appreciated that these common smoke detector test kits may not be as controlled as would otherwise be ideal (e.g., there may be a wide range of CO released). By dual calibrating the CO sensor and the VOC sensor more accurate calibration of the VOC sensor may be possible, even while using the non-precise smoke detector test kits. For example, the CO sensor (which is an extremely precise sensor) may be used to verify the amount of CO that the VOC sensor is being exposed to and thus enable a more precise calibration of the VOC sensor.

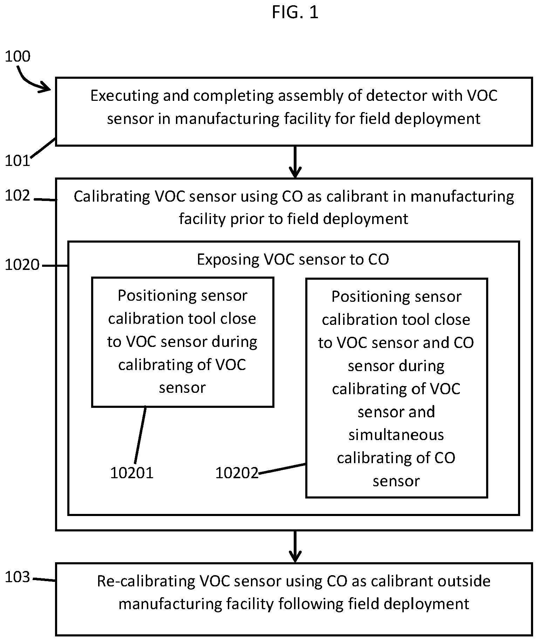

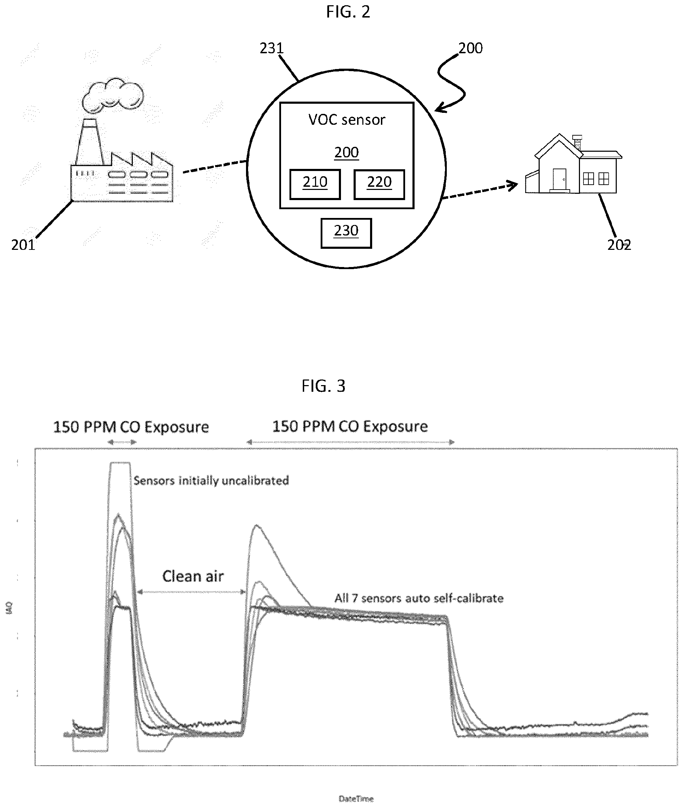

With reference to FIGS. 1 and 2, a method 100 of calibrating a detector 231 including a VOC sensor 200 and a CO sensor is provided. As shown in FIG. 1, the method 100 includes executing and completing an assembly of the VOC sensor 200 (see FIG. 2) in a manufacturing facility 201 (see FIG. 2) for field deployment (block 101) in a given space, such as the interior of a building or a house 202 (see FIG. 2). The method further includes calibrating the VOC sensor 200 using carbon monoxide (CO) as a calibrant in the manufacturing facility 201 prior to the field deployment (block 102) by exposing the VOC sensor 200 to the CO (block 1020). In addition, the method can include re-calibrating the VOC sensor 200 using the CO as the calibrant outside of the manufacturing facility 201 following the field deployment (block 103).

The VOC sensor 200 can include at least one of a metal oxide sensor 210 and an electrochemical sensor 220. The exposing of the VOC sensor 200 to the CO of block 1020 includes positioning a sensor calibration tool close to the VOC sensor 200 during the calibrating of the VOC sensor 200 (block 10201). The VOC sensor 200 is paired with the CO sensor 230 in the detector 231. The CO sensor 230 can be configured to determine a verified amount of CO the single detector 231 is exposed to, where the verified amount can be used to calibrate the VOC sensor 200.

With reference to FIG. 3, the calibrating of the VOC sensor of block 102 and the re-calibrating of the VOC sensor of block 103 can include exposing the VOC sensor 200 to the CO (having a concentration of about 400 PPM) for a relatively short exposure time of about 4-15 minutes. In some but not all cases, the calibrating of the VOC sensor of block 102 and the re-calibrating of the VOC sensor of block 103 can further include exposing the VOC sensor to the CO (having a concentration of about 150 PPM) for a relatively long exposure time following a delay after completion of the relatively short exposure time to confirm calibration status.

In accordance with embodiments, the re-calibrating of the VOC sensor 200 of block 103 can be periodic, regularly scheduled, or scheduled in response to a malfunction.

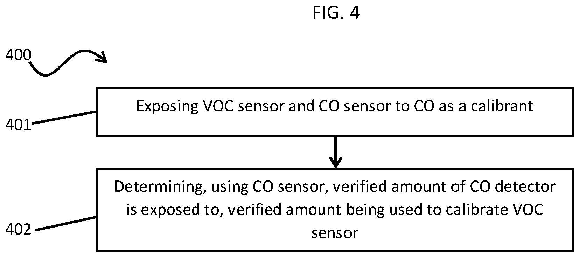

With reference to FIG. 4, a method 400 of calibrating a detector that includes a VOC sensor and a CO sensor, such as the detector 231 described above, is provided. As shown in FIG. 4, the method 400 includes exposing the VOC sensor and the CO sensor to CO as a calibrant (block 401) and determining, using the CO sensor, a verified amount of CO the detector is exposed to, the verified amount being used to calibrate the VOC sensor (block 402).

Technical effects and benefits of the present invention include the use of CO as a calibrant to thereby avoid the need for additional calibration systems to be installed in production of VOC systems in facilities that already perform CO calibration for CO alarms and detectors. Furthermore, systems that perform field calibration of CO can also be utilized to perform periodic field calibration of VOC sensors without the need for additional equipment and fulfil standards requirements for periodic calibration. Field calibration of VOC sensors can be offered as a service.

The corresponding structures, materials, acts, and equivalents of all means or step plus function elements in the claims below are intended to include any structure, material, or act for performing the function in combination with other claimed elements as specifically claimed. The description of the present disclosure has been presented for purposes of illustration and description, but is not intended to be exhaustive or limited to the technical concepts in the form disclosed. Many modifications and variations will be apparent to those of ordinary skill in the art without departing from the scope of the appended claims.

The embodiments were chosen and described in order to best explain the principles of the disclosure and the practical application, and to enable others of ordinary skill in the art to understand the disclosure for various embodiments with various modifications as are suited to the particular use contemplated.

While the preferred embodiments of the present invention have been described, it will be understood that those skilled in the art, both now and in the future, may make various improvements and enhancements which fall within the scope of the claims which follow.

## Claims

1. A method of calibrating a detector (231) comprising a volatile organic compound, VOC, sensor (200) that is sensitive to carbon monoxide, CO, and a CO sensor (230) that is previously calibrated, the method comprising:
assembling the detector (231) with the VOC sensor (200) for field deployment; and
calibrating the VOC sensor (200) using CO as a calibrant prior to the field deployment;
wherein the calibrating of the VOC sensor (200) comprises exposing the VOC sensor (200) to the CO;
wherein the exposing of the VOC sensor (200) to the CO comprises positioning a sensor calibration tool close to the VOC sensor (200) and the previously calibrated CO sensor (230) during exposure of an unknown quantity of CO to the VOC sensor (200) and the previously calibrated CO sensor (230); and
wherein the previously calibrated CO sensor (230) is used to measure a quantity of the CO by which the VOC sensor (200) is calibrated.

2. The method according to claim 1, wherein:
the VOC sensor (200) comprises at least one of a metal oxide sensor (210) and an electrochemical sensor (220).

3. The method according to claim 1 or 2, wherein the assembling of the VOC sensor (200) is executed and completed in a manufacturing facility (201).

4. The method according to claim 1, 2 or 3, wherein the CO sensor (230) is configured to determine a verified amount of CO the detector (231) is exposed to, the verified amount being used to calibrate the VOC sensor (200).

5. The method according to any of claims 1 to 4, wherein the calibrating of the VOC sensor (200) comprises exposing the VOC sensor (200) to the CO for at least a relatively short exposure time of about 4-15 minutes.

6. The method according to claim 5, wherein the CO has about a 400 PPM concentration during the relatively short exposure time.

7. The method according to any of claims 1 to 6, further comprising re-calibrating the VOC sensor (200) using the CO as the calibrant following the field deployment.

8. The method according to claim 7, wherein the re-calibrating is periodic, regularly scheduled or scheduled in response to a malfunction.

## Patentansprüche

1. Verfahren zum Kalibrieren eines Detektors (231), umfassend eine flüchtige organische Verbindung, VOC, einen Sensor (200), der empfindlich auf Kohlenmonoxid, CO ist, und einen CO-Sensor (230), der zuvor kalibriert wurde, wobei das Verfahren Folgendes umfasst:
Montieren des Detektors (231) mit dem VOC-Sensor (200) zur Feldausbringung; und
Kalibrieren des VOC-Sensors (200) unter Verwendung von CO als Kalibrator vor der Feldausbringung;
wobei das Kalibrieren des VOC-Sensors (200) Aussetzen des VOC-Sensors (200) dem CO umfasst;
wobei das Aussetzen des VOC-Sensors (200) gegenüber dem CO das Positionieren eines Sensorkalibrierungswerkzeugs nahe dem VOC-Sensor (200) und des zuvor kalibrierten CO-Sensors (230) während des Aussetzens einer unbekannten Menge von CO dem VOC-Sensor (200) und dem zuvor kalibrierten CO-Sensor (230) umfasst; und
wobei der zuvor kalibrierte CO-Sensor (230) verwendet wird, um eine Menge des CO zu messen, mit dem der VOC-Sensor (200) kalibriert wird.

2. Verfahren nach Anspruch 1, wobei:
der VOC-Sensor (200) mindestens einen von einem Metalloxidsensor (210) und einem elektrochemischen Sensor (220) umfasst.

3. Verfahren nach Anspruch 1 oder 2, wobei das Montieren des VOC-Sensors (200) in einer Fertigungseinrichtung (201) ausgeführt und abgeschlossen wird.

4. Verfahren nach Anspruch 1, 2 oder 3, wobei der CO-Sensor (230) konfiguriert ist, um eine verifizierte Menge von CO zu bestimmen, der der Detektor (231) ausgesetzt ist, wobei die verifizierte Menge verwendet wird, um den VOC-Sensor (200) zu kalibrieren.

5. Verfahren nach einem der Ansprüche 1 bis 4, wobei das Kalibrieren des VOC-Sensors (200) das Aussetzen des VOC-Sensors (200) dem CO für zumindest eine relativ kurze Aussetzungszeit von etwa 4 bis 15 Minuten umfasst.

6. Verfahren nach Anspruch 5, wobei das CO während der relativ kurzen Aussetzungszeit eine Konzentration von etwa 400 PPM aufweist.

7. Verfahren nach einem der Ansprüche 1 bis 6, ferner umfassend das Neukalibrieren des VOC-Sensors (200) unter Verwendung des CO als Kalibrator nach der Feldausbringung.

8. Verfahren nach Anspruch 7, wobei das Neukalibrieren als Reaktion auf eine Fehlfunktion periodisch, regelmäßig geplant oder geplant erfolgt.

## Revendications

1. Procédé d'étalonnage d'un détecteur (231) comprenant un capteur (200) de composé organique volatil, COV, sensible au monoxyde de carbone, CO, et un capteur de CO (230) préalablement étalonné, le procédé comprenant :
l'assemblage du détecteur (231) avec le capteur de COV (200) pour un déploiement sur le terrain ; et
l'étalonnage du capteur de COV (200) à l'aide de CO comme étalon avant le déploiement sur le terrain ;
dans lequel l'étalonnage du capteur de COV (200) comprend l'exposition du capteur de COV (200) au CO ;
dans lequel l'exposition du capteur de COV (200) au CO comprend le positionnement d'un outil d'étalonnage de capteur à proximité du capteur de COV (200) et du capteur de CO (230) précédemment étalonné pendant l'exposition d'une quantité inconnue de CO au capteur de COV (200) et au capteur de CO (230) précédemment étalonné ; et
dans lequel le capteur de CO (230) préalablement étalonné est utilisé pour mesurer une quantité de CO par laquelle le capteur de COV (200) est étalonné.

2. Procédé selon la revendication 1, dans lequel :
le capteur de COV (200) comprend au moins l'un d'un capteur d'oxyde métallique (210) et d'un capteur électrochimique (220).

3. Procédé selon la revendication 1 ou 2, dans lequel l'assemblage du capteur de COV (200) est exécuté et terminé dans une usine de fabrication (201).

4. Procédé selon la revendication 1, 2 ou 3, dans lequel le capteur de CO (230) est configuré pour déterminer une quantité vérifiée de CO à laquelle le détecteur (231) est exposé, la quantité vérifiée étant utilisée pour étalonner le capteur de COV (200).

5. Procédé selon l'une quelconque des revendications 1 à 4, dans lequel l'étalonnage du capteur de COV (200) comprend l'exposition du capteur de COV (200) au CO pendant au moins un temps d'exposition relativement court d'environ 4 à 15 minutes.

6. Procédé selon la revendication 5, dans lequel le CO a une concentration d'environ 400 PPM pendant le temps d'exposition relativement court.

7. Procédé selon l'une quelconque des revendications 1 à 6, comprenant également le réétalonnage du capteur de COV (200) en utilisant le CO comme étalon après le déploiement sur le terrain.

8. Procédé selon la revendication 7, dans lequel le réétalonnage est périodique, programmé régulièrement ou programmé en réponse à un dysfonctionnement.
